# EUROPÄISCHE PATENTSCHRIFT

(11) **EP 0 813 864 B1**
(45) Veröffentlichungstag und Bekanntmachung des Hinweises auf die Patenterteilung: **08.05.2002**
(21) Anmeldenummer: 97109461.0
(22) Anmeldetag: 11.06.1997
(51) Int. Cl.: A61K 9/46

(54) **Brausesystem für Brausetabletten und -granulate, sowie Verfahren zur Herstellung des Brausesystems**
Effervescent system for effervescent tablets and granulates and processes for preparing the effervescent system
Système effervescent pour des comprimés et des granulés effervescents ainsi que des procédés de fabrication du système effervescent

(30) Priorität: 20.06.1996 CH 154196
(43) Veröffentlichungstag der Anmeldung: 29.12.1997
(73) Patentinhaber: Gergely, Gerhard, Dr., A-1050 Wien (AT)
(72) Erfinder: Gergely, Gerhard Dr., 1050 Wien (AT); Gergely, Irmgard, 1050 Wien (AT); Gergely, Thomas Dr., 1050 Wien (AT); Gergely, Stefan Dr., 1050 Wien (AT)
(74) Vertreter: Büchel, Kurt F., Dr.

(56) Entgegenhaltungen:
- GB-A- 2 174 004
- US-A- 3 105 792
- US-A- 3 984 527
- DATABASE WPI Week 8009 Derwent Publications Ltd., London, GB; AN 80-15381c XP002025998 & JP 55 007 246 A (KANEBO KK) , 19.Januar 1980

## Beschreibung

Die Erfindung betrifft ein Brausesystem für Brausetabletten und -granulate nach dem Oberbegriff des Anspruches 1, sowie ein Verfahren zur Herstellung des Brausesystems nach dem Oberbegriff des Anspruches 5. Ein solches Brausesystem ist aus der US-A-3,105.792 bekannt geworden, deren Inhalt hiemit als im Rahmen der vorliegenden Beschreibung geoffenbart gilt, und wonach Alkalihydrogencarbonat kurze Zeit auf höhere Temperaturen oder längere Zeit auf weniger hohe Temperaturen - z.B. ausgebreitet auf grossen, luftüberströmten Tassen - erhitzt wird.

Bekannterweise ist die Stabilität von Natriumhydrogencarbonat einerseits gegen organische Säuren, z.B. Zitronensäure oder Weinsäure, dadurch vermindert, dass es ohne besondere Massnahmen aufgrund von immer vorhandener Restfeuchtigkeit zu Reaktionen zwischen Natriumhydrogencarbonat und der Säure kommt. Andererseits neigt das Natriumhydrogencarbonat schon für sich allein bei auch nur geringfügig erhöhten Temperaturen nach der Formel

2NaHCO₃ = Na₂CO₃ + CO₂ + H₂O

zu thermischer Zersetzung.

Man kann nun die Reaktion mit der Säure - aber eben nur teilweise - verhindern, indem man sie passiviert, d.h. oberflächlich zu einer Teilreaktion mit einem Carbonat oder Hydrogencarbonat bringt. Eine weitere Verbesserung der Stabilität ergibt sich, wenn die Natriumhydrogencarbonat-Partikeln selbst oberflächlich partiell zu Natriumcarbonat umgewandelt werden wie dies in der US-A-3,105.792 beschrieben ist. Dabei wird das entstehende Wasser wenigstens zum Teil gleich als Kristallwasser in das Natriumcarbonat eingebaut, das aber seiner Natur nach wiederum reaktionsfähig ist.

Eine genauere Untersuchung des in der US-A-3,105.792 beschriebenen Verfahrens zeigte jedoch, dass mit Alkalihydrogencarbonat-Partikeln, die mit Alkalicarbonat überzogen sind, zwar hinsichtlich der Stabilität des Brausesystems und allfälliger Wirkstoffe, z.B. der Acetylsalicylsäure, eine Verbesserung gegenüber handelsüblichen Alkalihydrogencarbonat-Partikeln erzielt werden konnte. Der Grad der Verbesserung war allerdings immer noch unbefriedigend, insbesondere bei längeren Lagerzeiten und/oder höheren Temperaturen, wie sie in heisseren Ländern durchaus auftreten können.

Bei der Erforschung der Ursachen für dieses unbefriedigende Verhalten wurde die Feststellung gemacht, dass offensichtlich das bei der Erwärmung des Alkalihydrogencarbonats freiwerdende Wasser nicht oder zumindest nicht vollständig entfernt werden konnte, sondern in das an der Oberflächenschicht der Partikel gebildete Alkalicarbonat als Kristallwasser eingebaut wird, das bei den angegebenen Reaktionstemperaturen und -zeiten nicht entfernbar ist und späterhin zu Sekundärreaktionen führt. Außerdem wäre es wichtig, daß das entstehende Alkalicarbonat an der Oberfläche der Alkalihydrogencarbonatkristalle gleichmässig verteilt ist, was offensichtlich durch die Erwärmung auf Tassen, selbst mit Hilfe von einigen Malen Umschaufeln, nur unzureichend erzielt werden kann.

Die Stabilitätsmessungen wurden nach zwei verschiedenen Gesichtspunkten durchgeführt, und zwar einmal durch die Bestimmung der CO₂-Entwicklung unter Wärmeeinwirkung, zum anderen durch die Auswahl eines sensiblen Brausesystems wie z.B. eines solchen mit Acetylsalicylsäure und die Bestimmung der Menge der unter dem Einfluss von Wärme und der Restfeuchtigkeit aus Acetylsalicylsäure gebildeten freien Salicylsäure mittels HPLC. Die Acetylsalicylsäure reagiert nämlich auch selbst mit dem Natriumhydrogencarbonat, wodurch unstabile Systeme relativ schnell sichtbar werden. Die CO₂-Entwicklung unter Wärmeeinwirkung wird wie folgt gemessen:

In einer gasdichten Messanordnung wird die Entwicklung von CO₂ aus einem Brausesystem unter Temperaturbelastung adiabatisch gemessen. Dies gibt Aufschluss über die Stabilität der Formulierung bei längerer Lagerung bei Raumtemperatur. Die Bestimmung ist sowohl ein Mass für das eventuell vorhandene Restwasser in der Brausebasis als auch für den Ablauf einer Abbaureaktion des Wirkstoffes (z.B. Acetylsalicylsäure), bei der Wasser gebildet wird, das in weiterer Folge wieder CO₂ freisetzt.

Die Probe (ca. 200 g Tabletten oder Brausegranulat) wird in einer dicht schliessenden Messingbombe in einem Wasserbad 20 min lang bei 45°C thermostatisiert. Nach dieser Zeit wird die Bombe mit der Messanordnung verbunden. Diese besteht aus einem mit Silikonöl gefüllten U-Rohr, das mit einem Magnetventil gasdicht verschlossen ist. Durch das entstehende CO₂-Gas verändert sich der Meniskus der Sperrflüssigkeit. Sobald dieser unter ein vorgewähltes Niveau gedrückt wird, schaltet ein Lichtschranken das Ventil und der Messvorgang beginnt von neuem (= 1 Impuls; die gemessenen Gasvolumina pro Impuls betragen, je nach gewählter Einstellung, zwischen 10 und 50 µl). Die Schaltimpulse des Ventils werden registriert.

Die Gesamtzahl der Impulse über die Messdauer entspricht der Gesamtmenge an entstandenem CO₂, der Abstand zwischen den Impulsen bzw. die Impulse pro Zeitintervall (z.B. 10 min) geben Aufschluss über das Verhalten des Systems; die Funktion n=f(Δt) konvergiert bei einem stabilen Produkt gegen 0.

Dabei zeigte es sich, dass aus einer acetylsalicylsäurehältigen Brausetablette mit unbehandeltem Natriumhydrogencarbonat bei 45°C schon nach wenigen Stunden 5 bis 10 Gew.% der Acetylsalicylsäure in freie Salicylsäure umgewandelt waren; mit nach der eingangs erwähnten US-A modifiziertem Natriumhydrogencarbonat waren es aber immer noch mehr als 1, meist etwa 5 Gew.%!

Die Erfindung hat sich daher die Aufgabe gestellt, ein Brausesystem gemäß dem Kennzeichen des Anspruches 1 zu schaffen, das eine um möglichst eine Grössenordnung bessere Stabilität hinsichtlich CO₂-Entwicklung und Acetylsalicylsäure-Abbau aufweist als das bekannte Brausesystem. Ein solches wird nun erfindungsgemäss erstmals durch die Kristallwasserfreiheit der Alkalicarbonat-Schichte vorgeschlagen. Die Herstellung gelang erstmals in überraschender Weise durch die Anwendung von Vakuum wie im Kennzeichen des Anspruches 5 beschrieben, insbesondere durch besonders leistungsstarke Pumpen, die das während der Wärmebehandlung bzw. aus der Konversion des Alkalihydrogencarbonates zum Alkalicarbonat gebildete Reaktionswasser sofort in den Dampfzustand übergehen lassen und abführen, bevor es als Kristallwasser in die gebildete Alkalicarbonat-Schichte eingebaut werden kann. Derart behandelte Alkalihydrogencarbonat-Partikel haben auch eine im wesentlichen zusammenhängende Carbonatschichte und ergeben unter den vorher angeführten Messbedingungen nur mehr weniger als 0.5, meist etwa 0.1 bis 0.2 Gew.% freie Salicylsäure.

Vorteilhafte Weiterbildungen der Erfindung sind in den Kennzeichen der abhängigen Ansprüche beschrieben.

Da die thermische Konvektion bei hohem Vakuum ausserordentlich schlecht ist, empfiehlt es sich, die Mantelheizung mit höheren Temperaturen von etwa 100°C bis etwa 120°C anzusetzen. Das Natriumhydrogencarbonat beginnt zwischen 80 und 100°C an seiner Oberfläche in Natriumcarbonat überzugehen, wobei gelegentlich stürmische thermische Zersetzungen auftreten können, wobei das sich entwickelnde CO₂ und der Wasserdampf das Vakuum - auch einer starken Pumpe - um 20 - 30 mbar ansteigen lassen können.

Ganz besonders wichtig für das Verfahren ist es, daß bei erreichtem gewünschtem Konversionsgrad der Mantel sofort gekühlt wird und auch die Abkühlung der entstandenen Mischkristalle ebenfalls unter hohem Vakuum durchgeführt wird, sodass ein Beenden des Vorganges nie über 60°C, vorzugsweise nie über 50°C Produkttemperatur stattfinden sollte. Erst danach soll die Masse aus dem Vakuumkessel entfernt werden.

Man kann den Konversionszustand auch sehr leicht unter dem Mikroskop im aufscheinenden, aber auch im durchscheinenden Licht feststellen, da die entstandenen Kristalle im aufscheinenden Licht gleichmässig trüb und im durchscheinenden Licht undurchsichtig geworden sind.

Setzt man solche Natriumhydrogencarbonate in Brausetablettenformeln mit beispielsweise Aspirin ein, dann ist es unter Umständen möglich, sogar mit unbehandelter Zitronensäure - ohne besondere Passivierungsmassnahmen - stabile Tabletten zu erreichen.

Ein weiterer Vorteil dieser Art konvertierten Natriumhydrogencarbonats besteht darin, daß durch die Gitterstörungen in den Kristallstrukturen starke elektrische Bindungskräfte entstehen, wodurch sich beim Pressen harte Tabletten erzielen lassen.

Der erfindungsgemässe Effekt kann theoretisch wie folgt erläutert werden: das Verfahren zur Teilkonversion von Natriumhydrogencarbonat ohne Vakuum in Natriumcarbonat nach der US-A-3,105.792 ist eigentlich nichts wesentlich anderes als der Zusatz von Natriumcarbonat.

Wird aber erfindungsgemäss auf den Alkalihydrogencarbonat-Kristallen die äussere Schicht in wasserfreies Natriumcarbonat umgewandelt, dann wird das bei thermischer Reaktion des Natriumhydrogencarbonats im Inneren während der Lagerung der Tablette gebildete H₂O und CO₂ sofort von der angrenzenden Schicht Natriumcarbonat wieder in Natriumhydrogencarbonat verwandelt. Trockenes Hydrogencarbonat ist aber weit weniger für die thermische Zersetzung anfällig als bei Anwesenheit von auch nur Spuren von Feuchtigkeit.

Dieser Vorgang kann hin und her pendeln und sichert das ganze System in stabiler Form ab. Er ist vergleichbar mit der Ausfällung des schwerer löslichen Natriumhydrogencarbonats durch Einleiten von Kohlensäuregas in Lösungen von Natriumcarbonat, oder mit der Bildung des Sesquicarbonats (Trona, NaHCO₃.Na₂CO₃.2H₂O), das in der Natur als stabile Form zwischen Natriumhydrogencarbonat und Natriumcarbonat (mit zwei Molekülen Kristallwasser!) in den Salzseen entsteht. Ein ähnlicher Vorgang ergibt sich nun im Pendeln zwischen innerer und äusserer Schicht des Alkalihydrogencarbonat-Kristalls, wo Trona-ähnliche Vorgänge als Endprodukt des Gleichgewichts der Reaktion geschehen.

Ist aber das Natriumcarbonat nicht trocken und/oder enthält es bereits Kristallwasser, dann kann dieser Vorgang nicht oder nur teilweise stattfinden, da ein Natriumcarbonat, das bereits Kristallwasser enthält, zu dieser Reaktion nicht mehr fähig ist. Voraussetzung für die stabile Struktur derartiger Gebilde ist daher die völlige Abwesenheit von Feuchtigkeit und auch besonders von Kristallwasser.

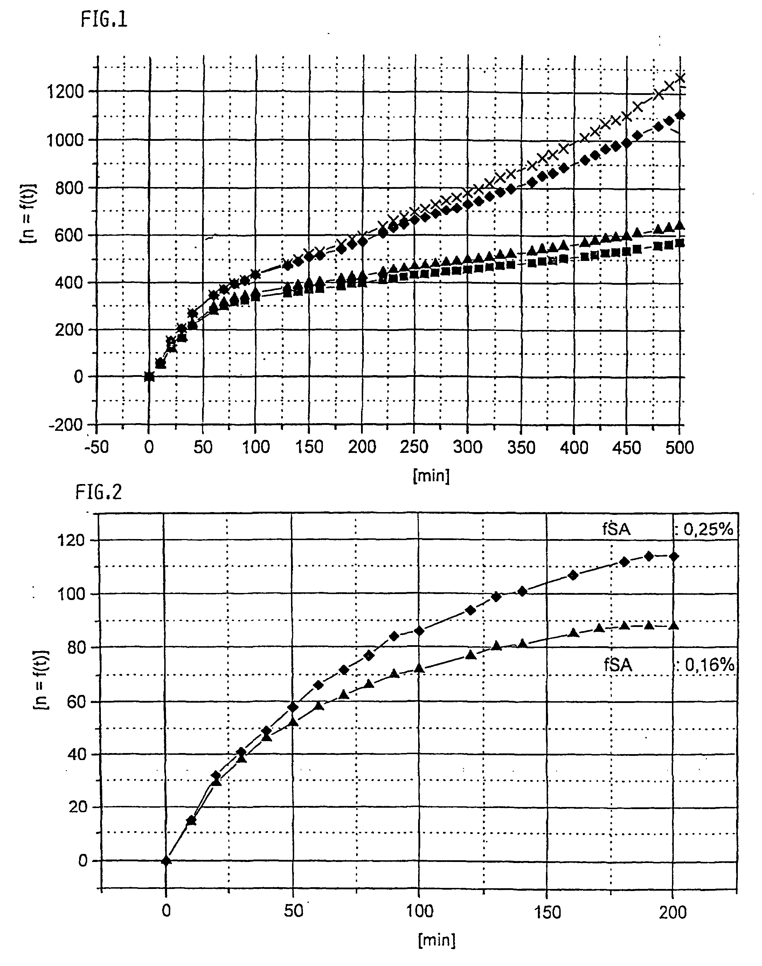

Die Erfindung wird anhand der Zeichnung beispielhaft näher erläutert. Fig.1 zeigt die CO₂-Entwicklung eines Produktes nach der US-A-3,105.792 zum Stand der Technik **(Beispiel 1):**

Ein Granulierkessel mit einer Manteltemperatur von 110°C wird mit 8 kg mittelgrobem Natriumhydrogencarbonat (60-65 Gew.% zwischen 0,1 und 0,2 mm) befüllt und unter Luftdurchleiten 3 Stunden lang erwärmt, bis die Produkttemperatur 100°C überschritten hat. Anschliessend wird während 2 Stunden auf 55°C abgekühlt, und zwar ein Teil 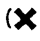 und ◆) an der Raumluft, ein anderer Teil (▲ und ■ ) im Kessel unter Luftdurchleiten bei geringem Unterdruck. Es ergibt sich ein Umsetzungsgrad von 6%.

Jeweils 1000 Gew.teile des so behandelten Natriumhydrogencarbonats werden mit 750 Gew.teilen Zitronensäure, 550 Gew.teilen Acetylsalicylsäure und 50 Gew. teilen Coffein zu je 2,3 g schweren Tabletten gepresst, die dann dem vorerwähnten Test zur Bestimmung der CO₂-Entwicklung unterworfen werden. Die Kurven der CO₂-Entwicklung finden sich in Fig.1, und zwar jeweils zwei Parallel-Versuche mit Abkühlung an der Raumluft 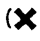 und ◆) mit 5.27 % freier Salicylsäure, bzw. im Kessel unter Luftdurchleiten bei geringem Unterdruck (▲ und ■ ) mit 1.88 % freier Salicylsäure, was immerhin schon eine wenn auch geringe Verbesserung bedeutet.

### Beispiel 2:

Der Vorgang nach Beispiel 1 wird wiederholt mit dem Unterschied, dass der Granulierkessel ein Vakuumkessel ist, an den eine Pumpe mit 100 m³/h Nennsaugvermögen angeschlossen ist. Es wird auf 5 mbar evakuiert; das Vakuum wird auch während der Behandlung und der Abkühlung immer unter 20 mbar gehalten.

Selbst mit einer getrockneten, aber nicht oberflächlich passivierten Zitronensäure ergab eine Bestimmung der freien Salicylsäure 0,25%, mit einer oberflächlich passivierten Zitronensäure 0,16%. Die CO₂-Entwicklung war, wie aus Fig.2 ersichtlich, in beiden Fällen um fast eine Grössenordnung geringer als nach Beispiel 1.

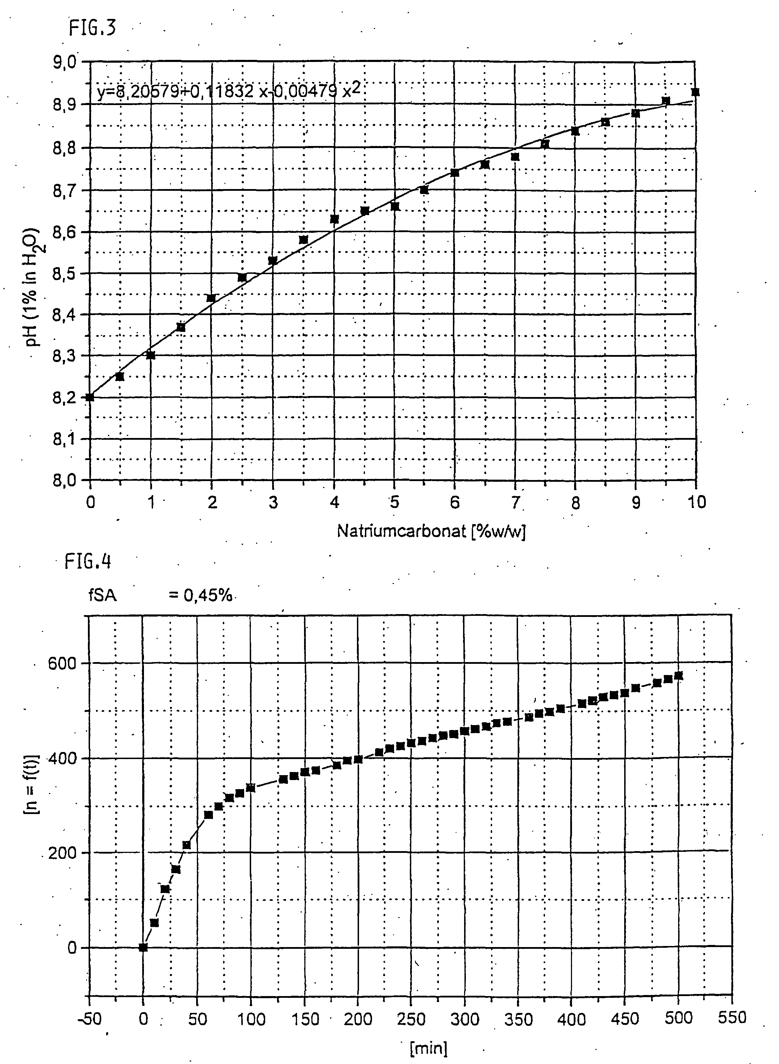

Der Umsetzungs- (Konversions)grad von Natriumhydrogencarbonat zu Natriumcarbonat kann näherungsweise sehr einfach durch die Messung des pH-Wertes einer 1%igen Lösung bestimmt werden (Fig.3).

Anstelle von Natrium- kann erfindungsgemäss mit Erzielung desselben positiven Effektes auch Kaliumhydrogencarbonat behandelt werden.

Die Korngrösse spielt in beiden Fällen keine gravierende Rolle; feine Pulver bis zu gröberen Kristallen, z.B. von 0.3 mm, konnten erfolgreich behandelt werden, wenn die Umsetzung zu mindestens 1, vorzugsweise 2 bis 4 Gew. %, jedoch maximal bis 10, vorzugsweise maximal bis zu 8 Gew.% getrieben wird. Unter 1 % wird die Schutzwirkung gefährlich niedrig; über 10% hat man weil Natriumcarbonat alkalisch reagiert - unter Umständen Probleme mit alkaliempfindlichen Wirkstoffen. Feinere Korngrössen werden; da sie eine grössere Oberfläche pro Gewicht haben, zur Erzielung einer gleichmässigen Schichtdicke eher mit den höheren Prozentsätzen innerhalb des angegebenen Bereiches umgesetzt.

Die Vakuumpumpe sollte ein Nennsaugvermögen von wenigstens 50, vorzugsweise wenigstens 100 m³/h haben.

### Beispiel 3 (Fig.4 bis 8):

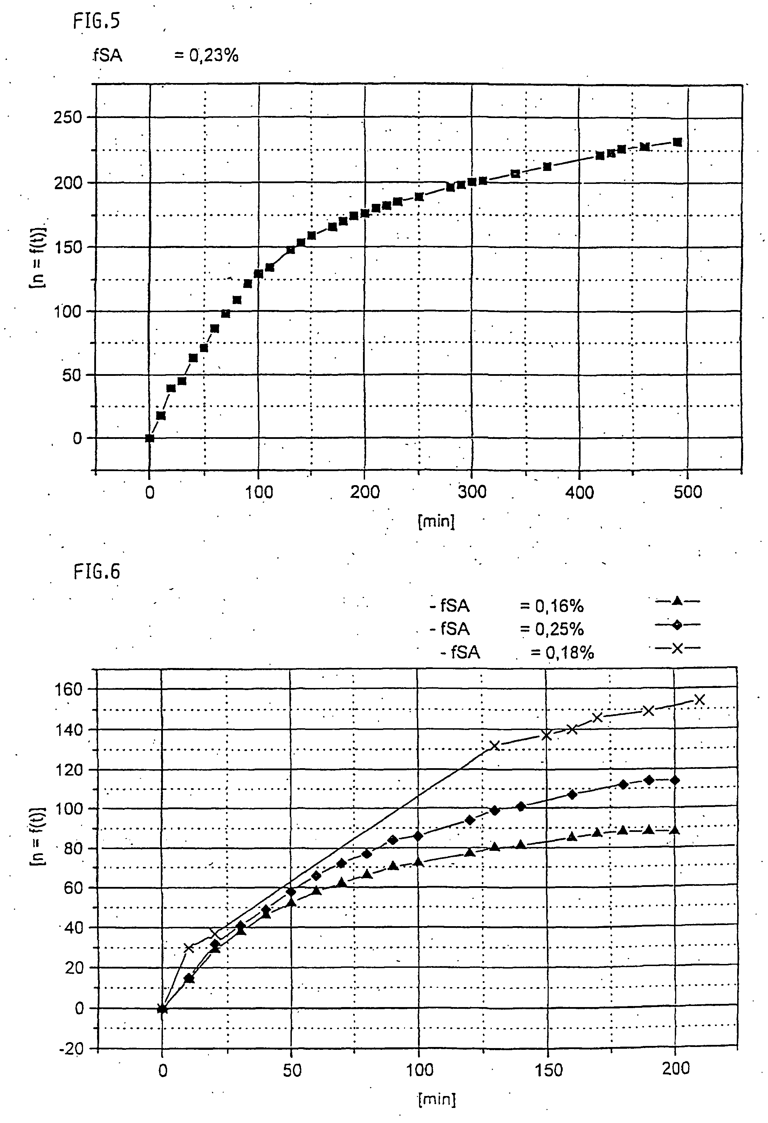

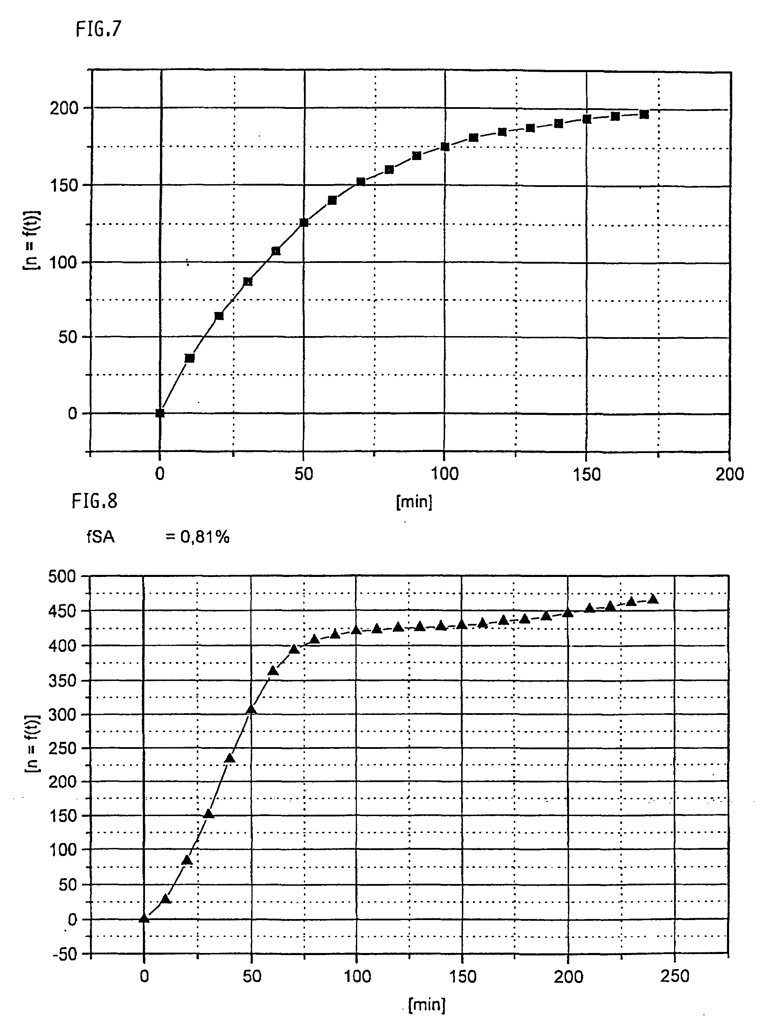

Die Fig.4 bis 7 zeigen einige weitere Kurvenverläufe (auf der Abszisse die Zeit in Minuten, auf der Ordinate die Impulszahl) der CO₂-Entwicklung erfindungsgemässer, analog zu Beispiel 2 hergestellter Brausesysteme, jeweils mit Angabe der entstandenen freien Salicylsäure fSA, und zwar Fig.4 mit einem nur zu 3,5% konvertierten, feinkörnigen Natriumhydrogencarbonat und getrockneter Zitronensäure, Fig.5 mit einem zu 3,4% konvertierten, mittelgroben Natriumhydrogencarbonat und passivierter Zitronensäure. Geht man mit der Konversion auf 8% (Fig.6, drei Parallel-Beispiele) oder gar auf 9.5% (Fig.7), wird sowohl die CO₂-Entwicklung wie auch die Bildung freier Salicylsäure noch weiter reduziert.

Demgegenüber wird mit einem ohne Vakuum zu 6% konvertierten, mittelgroben Natriumhydrogencarbonat mehr als die doppelte Menge CO₂ gebildet (Fig.8).

Die Erfindung ist nicht auf die angeführten Beispiele eingeschränkt. Sie ist wie erwähnt, gegebenenfalls mit geeigneter, jedem einschlägigen Fachmann geläufiger Adaption, auch für andere Wirkstoffe geeignet, die empfindlich gegen einen Bestandteil des Brausesystems, bzw. insbesondere gegen Restfeuchtigkeit sind.

## Patentansprüche

1. Brausesystem für Brausetabletten und/oder Brausegranulat, enthaltend einerseits Partikel einer festen, essbaren, organischen Säure, andererseits Partikel wenigstens eines Alkalihydrogencarbonates, die von einer Schicht Alkalicarbonat überzogen sind, **dadurch gekennzeichnet, dass** die Alkalicarbonat-Schichte im wesentlichen zusammenhängend, sowie trocken und kristallwasserfrei ist.

2. Brausesystem nach Anspruch 1, **dadurch gekennzeichnet, dass** die Carbonatschichte wenigstens 1, vorzugsweise 2 bis 4, höchstens jedoch 10, vorzugsweise höchstens 8 Gew.% der Hydrogencarbonat-Partikel ausmacht.

3. Brausesystem nach Anspruch 1 oder 2, **dadurch gekennzeichnet, dass** die Säurepartikel, vorzugsweise Zitronensäurepartikel, in an sich bekannter Weise durch Anreaktion mit - vorzugsweise etwa 20 bis etwa 40 Gew.% - wenigstens eines Carbonats und/oder Hydrogencarbonats abgedeckt sind.

4. Brausesystem nach einem der vorhergehenden Ansprüche, mit wenigstens einem Wirkstoff, der mit wenigstens einem der Bestandteile des Brausesystems unter Abbau reagiert, wie z.B. mit Acetylsalicylsäure, **dadurch gekennzeichnet, dass** nach einer 6-stündigen Behandlung bei 45°C weniger als 1%, vorzugsweise weniger als 0.5%, insbesondere höchstens etwa 0.2% des Wirkstoffes abgebaut sind.

5. Verfahren zur Herstellung eines Brausesystems nach einem der Ansprüche 1 bis 3, **dadurch gekennzeichnet, dass** die Alkalihydrogencarbonat-Partikel in einem Vakuumkessel unter schwingendem Mischen bei weniger als 40, vorzugsweise weniger als 20, insbesondere weniger als 10 mbar bei über 60°C, bevorzugt zwischen 80°C und 120°C, insbesondere bei etwa 100°C, wärmebehandelt und danach unter Aufrechterhaltung des Vakuums auf unter 60°C, vorzugsweise auf unter 50°C, abgekühlt werden.

6. Verfahren nach Anspruch 5, **dadurch gekennzeichnet, dass** der Mantel des Vakuumkessels vor der Befüllung mit den Alkalihydrogencarbonat-Partikeln auf eine Temperatur von etwa 100°C bis etwa 120°C vorgewärmt wird.

7. Verfahren nach einem der Ansprüche 5 oder 6, **dadurch gekennzeichnet, dass** das Vakuum mit Hilfe einer Vakuumpumpe eingestellt und aufrechterhalten wird, die ein Nennsaugvermögen von mindestens 50, vorzugsweise mindestens 100 m³/h aufweist, so dass auch während des Behandlungsvorganges das Vakuum nie über 40, vorzugsweise nie über 20 mbar ansteigt.

## Claims

1. Effervescent system for effervescent tablets and/or effervescent granules, containing, on the one hand, particles of a solid, edible, organic acid and, on the other hand, particles of at least one alkali metal bicarbonate which are coated with an alkali metal carbonate, **characterized in that** the alkali metal carbonate coat is substantially continuous and is dry and free of water of crystallization.

2. Effervescent system according to Claim 1, **characterized in that** the carbonate coat accounts for at least one, preferably 2 to 4, but not more than 10, preferably not more than 8, % by weight of the bicarbonate particles.

3. Effervescent system according to Claim 1 or 2, **characterized in that** the acid particles, preferably citric acid particles, are covered in a manner known per se by superficial reaction with - preferably about 20 to about 40% by weight of - at least one carbonate and/or bicarbonate.

4. Effervescent system according to any of the preceding Claims, comprising at least one active substance which reacts with at least one of the components of the effervescent system with degradation, such as, for example, with acetylsalicylic acid, **characterized in that**, after treatment for 6 hours at 45°C, less than 1%, preferably less than 0.5%, in particular not more than about 0.2%, of the active substance has been degraded.

5. Process for preparing an effervescent system according to any of Claims 1 to 3, **characterized in that** the alkali metal bicarbonate particles are heat-treated in a vacuum vessel with vibratory mixing at less than 40, preferably less than 20, in particular less than 10, mbar at over 60°C, preferably between 80°C and 120°C, in particular at about 100°C, and then cooled to below 60°C, preferably to below 50°C, while maintaining the vacuum.

6. Process according to Claim 5, **characterized in that** the jacket of the vacuum vessel is preheated to a temperature of about 100°C to about 120°C prior to the filling with the alkali metal bicarbonate particles.

7. Process according to either of Claims 5 and 6, **characterized in that** the vacuum is established and maintained with the aid of a vacuum pump which has a rated throughput of at least 50, preferably at least 100, m³/h, so that the vacuum never increases above 40, preferably never above 20, mbar even during the treatment procedure.

## Revendications

1. Système effervescent pour des comprimés effervescents et / ou pour des granulés effervescents contenant, d'une part, des particules d'un acide organique alimentaire solide et, d'autre part, des particules d'au moins un hydrogénocarbonate de métal alcalin, qui sont couvertes d'une couche de carbonate de métal alcalin, **caractérisé en ce que** la couche de carbonate de métal alcalin est sensiblement continue, ainsi que sèche et exempte d'eau de cristallisation.

2. Système effervescent selon la revendication 1, **caractérisé en ce que** la couche de carbonate constitue au moins 1 % en poids, de préférence de 2 à 4 % en poids, au maximum 10 % en en poids, mais de préférence, au maximum 8 % en poids, des particules d'hydrogénocarbonate de métal alcalin.

3. Système effervescent selon la revendication 1 ou la revendication 2, **caractérisé en ce que** sur les particules d'acide, de préférence des particules d'acide citrique, on réalise par réaction et d'une manière connue, un revêtement d'au moins un carbonate et / ou un hydrogénocarbonate, appliqué, de préférence, à raison d'environ 20 à environ 40 % en poids.

4. Système effervescent selon l'une des revendications précédentes, comprenant au moins un agent actif, comme par exemple de l'acide acétyle salicylique, qui réagit en se décomposant avec au moins une partie constitutive du système effervescent, **caractérisé en ce qu'**après un traitement de 6 heures à 45 °C, moins de 1 % en poids, de préférence moins de 0,5 % en poids et surtout au maximum environ 0,2 % en poids de l'agent actif sont décomposés.

5. Procédé de préparation d'un système effervescent selon l'une des revendications 1 à 3, **caractérisé en ce que** les particules d'hydrogénocarbonate de métal alcalin sont soumises à un traitement thermique dans des conditions de mélange par oscillations, dans un récipient mis sous un vide inférieur à 40 mbars, de préférence inférieur à 20 mbars et surtout inférieur à 10 mbars, à une température dépassant 60 °C, de préférence comprise entre 80 °C et 120°C et, en particulier, d'environ 100 °C, et **en ce qu'**ensuite, ces particules sont refroidies en maintenant le vide, à une température inférieure à 60 °C et, de préférence, inférieure à 50 °C.

6. Procédé selon la revendication 5, **caractérisé en ce que** l'enceinte du récipient mis sous vide est préchauffée à une température dans la plage allant d'environ 100 °C à environ 120 °C, avant son remplissage avec les particules d'hydrogénocarbonate de métal alcalin.

7. Procédé selon la revendication 5 ou la revendication 6, **caractérisé en ce que** le vide est atteint et maintenu à l'aide d'une pompe à vide ayant une capacité d'aspiration nominale d'au moins 50 m³ et, de préférence, d'au moins 100 m³/h, de manière à ce que durant le processus de traitement, la pression résiduelle ne dépasse jamais 40 et, de préférence, 20 mbars.
